# EUROPÄISCHE PATENTSCHRIFT

(11) **EP 2 015 721 B1**
(45) Veröffentlichungstag und Bekanntmachung des Hinweises auf die Patenterteilung: **26.08.2009**
(21) Anmeldenummer: 07724646.0
(22) Anmeldetag: 27.04.2007
(51) Int. Cl.: A61F 13/00, A61F 13/62, A44B 18/00, D03D 15/08

(54) **MEDIZINISCHES FLÄCHENGEBILDE**
MEDICAL PLANAR STRUCTURE
STRUCTURE PLANE À USAGE MÉDICAL

(30) Priorität: 11.05.2006 DE 102006022971
(43) Veröffentlichungstag der Anmeldung: 21.01.2009
(73) Patentinhaber: Karl Otto Braun GmbH & Co. KG., 67752 Wolfstein (DE)
(72) Erfinder: LANGEN, Günter, 67752 Wolfstein (DE)
(74) Vertreter: Langöhrig, Angelika Beate
(86) Internationale Anmeldenummer: PCT/EP2007/003716
(87) Internationale Veröffentlichungsnummer: WO 2007/131605

(56) Entgegenhaltungen:
- WO-A-02/38096
- WO-A-20/04058122
- DE-A1- 2 240 945
- GB-A- 1 080 653
- US-A- 5 015 251
- US-A1- 2002 022 108

## Beschreibung

Die Erfindung betrifft ein medizinisches haftfähiges Flächengebilde, vorzugsweise in Form einer Binde oder Bandage, das dehnungselastisch ist und eine Dehnbarkeit zwischen 40 und 300 % aufweist, welches ein textiles Substrat umfasst, wobei das Substrat eine erste und eine zweite Seite besitzt und mindestens eine Kettfadenschar umfasst.

Im Stand der Technik ist es bekannt, dehnbare oder elastische medizinische Flächengebilde in Form von Geweben, Gewirken, Gestricken und Vliesen vorzusehen, wobei dehnungselastische Kettfäden aus hochgedrehten Baumwollfäden, Elastodien, Elastan, thermoplastischem Kautschuk oder texturierten Multifilamentgarnen aus Chemiefasern teilweise in Kombination mit anderen unelastischen Kettfäden und geeigneten Schussfäden sowie Vliesstoffen verarbeitet werden. Die in dieser Weise gefertigten Flächengebilde weisen parallel zu den Kettfäden eine Dehnungselastizität auf, d.h. das Flächengebilde kann durch Anwendung einer Zugkraft in Längsrichtung gedehnt werden und kehrt bei Wegnahme der Zugkraft durch den elastischen Rückzug mehr oder weniger wieder in den ungedehnten Ausgangszustand zurück. Um eine bessere Befestigung derartiger elastischer Binden am Körperteil zu erreichen, offenbart der Stand der Technik die Möglichkeit einer haftklebenden Beschichtung der Textiloberflächen unter Verwendung von Haftklebstoffen die kohäsive oder adhäsive Eigenschaften aufweisen. Derartige medizinische Flächengebilde besitzen die Fähigkeit, auf sich selbst zu haften, d. h. beim Anwickeln und Anpressen einer Binde haften beispielsweise die Bindenlagen aufeinander (Kontakthaftung), so dass ein rutschfester Verband entsteht.

Bei Verwendung von kohäsiven Klebemitteln haften die Bindenlagen nur auf sich selbst, aber nicht auf fremden Oberflächen wie Haut, Haaren, Kleidungsstücken. Im Gegensatz dazu haften adhäsive Klebemittel auch auf fremden Oberflächen, insbesondere auch auf der Haut. Dabei ist es bei kohäsiven Binden in der Regel üblich, Naturkautschuklatex als kohäsives Klebemittel zu verwenden, was jedoch den Nachteil besitzt, dass dieser Allergien auszulösen vermag.

So sind elastische Verbandmaterialien mit haftklebend beschichteter Oberfläche beispielsweise aus der EP 0 258 484 sowie der WO 00/68334 A1 bekannt, in der auch zahlreiche Beispiele für kohäsive und adhäsive Klebstoffe genannt sind. Diese beschreiben auch die Nachteile bekannter kohäsiver Binden auf Naturkautschuklatexbasis, nämlich mangelnde Licht-, Temperatur- und Alterungsbeständigkeit sowie die Anwesenheit von Proteinen und Peptiden mit allergenem Potential, die bei Anwendern und Patienten fallweise zu Kontaktallergien vom Soforttyp führen kann. Diesem Problem soll durch Verwendung eines Klebemittels auf Basis eines Schmelzklebstoffs in Form einer naturlatexfreien Kautschuk-Harz-Mischung mit definierten rheologischen Eigenschaften begegnet werden.

Nachteilig ist hier jedoch neben der komplizierten Verfahrens- und Beschichtungstechnik u. a. die Restklebrigkeit auf der Haut aufgrund von Harzzusätzen, d.h. ein zumindest partiell ausgeprägter adhäsiver Charakter des Klebeverhaltens. Darüber hinaus wird durch den Auftrag der hochviskosen Schmelzklebemasse die elastische Verformbarkeit bzw. maximale Dehnbarkeit des Trägertextils negativ beeinträchtigt. Die WO 2004/058122 beschreibt weiterhin eine medizinische Binde mit einer Depotfunktion zur Verabreichung von Wirkstoffen auf die Haut bzw. ein Körperteil. Die beschriebene Binde besteht aus einem flexiblen Substrat in Bandform, welches mit wenigen streifenförmigen Haftfolienbändern, die sich über die gesamte Länge bzw. Breite des Substrats erstrecken, ausgestattet ist. Die Haftstreifen verankern sich mit der Rückseite des Substrates, wobei die Flexibilität des Substrates negativ beeinträchtigt wird. Dabei sind die Haftstreifen bevorzugt in der Nähe der Enden des streifenförmigen Substrates angebracht, so dass keine gleichmäßig wirkende Haftung über die gesamte Fläche des Substrats erreicht wird.

Schließlich beschreibt die DE 198 54 320 textile Flächengebilde mit haftender Oberfläche, die durch Einarbeitung von bändchenförmigen Klettfäden direkt bei der Herstellung der textilen Flächengebilde durch Web- oder Wirktechnik gebildet wird. Auch hierbei erstrecken sich die Klettfäden über die gesamte Länge bzw. Breite des Flächengebildes, wodurch eine Blockade der dehnungselastischen Eigenschaften resultiert.

Die EP 950 363 beschreibt ein Verfahren zur Herstellung von gegossenen Flächenhaftverschlüssen in Gestalt von Polymerfolien, auf deren Oberfläche Haftmittel in Gestalt von Stengel- oder Hakenformen angebracht sind. Die Stengel können verschiedene Kopfformen, z.B. eine Pilzkopf-Form aufweisen, um eine bessere Verhakung mit dem Gegenstück des Flächenhaftverschlusses (Flauschteil) zu ermöglichen. Die Stengel und Haken können in unterschiedlichen Geometrien und Anordnungen auf der Oberfläche der Polymerfolien angebracht sein.

Die EP 723 406 sowie die WO 99/17631 beschreiben elastische Befestigungssysteme, bei denen die Hafthaken oder Haftstengel auf einem elastisch dehnbaren Substrat aus Naturkautschuk, thermoplastischem Kautschuk, Polyurethan-Elastomeren oder Metallocen-katalysierten Polyolefinen angebracht sind. Das Substrat in Gestalt von Folien, Schaumstoffen kann eine Dehnbarkeit von bis zu 100 % aufweisen und ist mit der Hafthaken-Folie laminiert. Derartige elastische Flächenverschlüsse werden vorteilhaft beim Verschließen von elastischen Windel- oder anderen Hygieneprodukten verwendet.

Die Dehnbarkeit von 40% bis 300 % entspricht einem üblichen Wert von elastischen Binden für medizinische Anwendungen wie die Stützung, Entlastung, Kompression von Körperteilen, wobei je nach maximaler Dehnbarkeit Binden mit Kurz-, Mittel-, bzw. Langzugeigenschaften unterschieden werden. Die Bestimmung der Dehnbarkeit erfolgt dabei gemäß DIN 61632 - 1985, wobei die maximale Dehnbarkeit einen elastischen und einen nicht-elastischen Anteil umfasst. Im Folgenden sind beide Aspekte in dem Begriff "dehnungselastisch" zusammengeführt und beinhalten somit sowohl rein dehnbare als auch rein elastische Eigenschaften sowie alle graduellen Übergänge dieser Eigenschaften.

Der Erfindung stellt sich nun die Aufgabe, ein dehnungselastisches medizinisches Flächengebilde mit Hafteigenschaften bereitzustellen, wobei das Flächengebilde keinerlei Haftung zur Haut und Haaren aufweisen soll, die potentielle Gefahr der Erzeugung von Allergien unterbunden wird und gleichzeitig die physikalischen Eigenschaften wie beispielsweise die dehnungselastische Eigenschaft des textilen Substrates möglichst wenig beeinträchtigt wird.

Die Erfindung löst diese Aufgabe durch ein gattungsgemäßes medizinisches Flächengebilde, bei dem auf der ersten Seite des Substrats eine erste mechanische Haftstruktur aufgebracht ist, die aus einer Vielzahl diskreter erster Haftelemente besteht und die erste mechanische Haftstruktur mit einer zweiten mechanischen Haftstruktur auf der der ersten Seite gegenüberliegenden zweiten Seite des Flächengebildes bzw. des Substrats in haftende Verbindung bringbar ist.

Dabei soll unter einer mechanischen Haftstruktur eine solche Struktur verstanden werden, bei der eine Haftung nicht mittels Haftklebstoffen mit adhäsiven oder kohäsiven Eigenschaften erfolgt, sondern mit einem mechanischen Verhaken oder Ineinandergreifen zweier Haftelemente einhergeht.

Dabei ist vorzugsweise vorgesehen, dass das medizinische Flächengebilde, das eine Länge L und eine Breite B aufweist, über die gesamte Länge L homogen dehnungselastisch ist. Hierbei ist unter einem homogen dehnungselastischen Flächengebilde einer Länge L und einer Breite B ein Flächengebilde zu verstehen, das über seine Länge L eine Dehnbarkeit D_{ges}. von X % mit 40 % < X % < 300 % besitzt und gleichzeitig die Dehnbarkeit Dₜₑᵢₗ jedes Teilbereiches des Flächengebildes mit der Länge 0,1 L annähernd denselben diskreten Wert zwischen 40 % und 300 % aufweist, wobei die Werte D_{ges}. und Dₜₑᵢₗ insgesamt maximal 15 % voneinander abweichen und der Teilbereich 0,1 L nicht kleiner als 20 cm sein darf. Die Bestimmung der Dehnbarkeit erfolgt dabei gemäß DIN 61632 - 1985, wobei die maximale Dehnbarkeit einen elastischen und einen nicht-elastischen Anteil umfasst.

Insbesondere weist ein erfindungsgemäßes medizinisches Flächengebilde eine Dehnbarkeit D_{ges} von 40 bis 200 % auf. Ganz besonders bevorzugt weist dieses Flächengebilde eine Dehnbarkeit D_{ges} von 60 bis 200 % und insbesondere 60 bis 160 % auf. Es kann jedoch auch vorgesehen sein, das das Flächengebilde eine Dehnbarkeit von 40 bis 120 % oder 120 bis 200 % aufweist, wobei besonders bevorzugt sämtliche dieser Flächengebilde auch homogen dehnungselastisch sind.

In einer weiteren besonders bevorzugten Ausführungsform weist das medizinische Flächengebilde eine Länge L und eine Breite B und eine Dehnbarkeit von 60 bis 160 % auf, wobei das Flächengebilde über die gesamte Länge L homogen dehnungselastisch ist.

Weiterhin vorzugsweise ist vorgesehen, dass die Haftung der Oberflächen des erfindungsgemäßen medizinischen Flächengebildes (gemessen als Haftkraft in cN/cm) an jeder Stelle des medizinischen Flächengebildes im Mittel gleich groß ist, d.h., dass die Haftkraft pro Zentimeter Breite des Flächengebildes über die Länge des Flächengebildes konstant innerhalb der Streugrenzen von ± 15 % ist. Die Haftkraft wird nach dem Verfahren der DIN EN 12242-1999 "Bestimmung der Abschälfestigkeit von Haftverschlüssen" ermittelt, wobei zum Überrollen und Verpressen der haftenden Textiloberflächen für eine Probenbreite größer 5 cm generell ein Metallzylindergewicht von 10 kg verwendet wird. Die beiden Schenkel der in dieser Weise vorbereiteten Messprobe werden in einer Zugprüfmaschine bei gleich bleibender Prüfgeschwindigkeit getrennt und die dazu notwendige Kraft über eine definierte Trennstrecke gemessen. Die Haftkraft ergibt sich aus dem arithmetischen Mittelwert der Trennkraft bezogen auf einen cm Probenbreite.

Auf diese Weise wird das technische Problem gelöst, ein dehnungselastisches Grundtextil mit einer Haftoberfläche auszustatten, ohne die physikalischen Eigenschaften des textilen Substrats, wie Flächengewicht, Dehnbarkeit, Flexibilität, Luftdurchlässigkeit, Tragekomfort etc. signifikant zu verändern und wobei eine Haftung nicht mittels Haftklebstoffen mit adhäsiver oder kohäsiver Eigenschaft erfolgt, sondern mittels eines mechanischen Verhakens oder Ineinandergreifens zweier Haftelemente unterschiedlicher Geometrie einhergeht.

Die Haftwirkung beruht nicht auf der Verwendung eines Klebemittels mit kohäsiver oder adhäsiver Haftwirkung, wie Polymerzubereitungen auf Basis von Naturkautschuk, Polyacrylat oder sonstigen Haftklebern, die ein allergenes oder hautreizendes Potential beinhalten. Das erfindungsgemäße Flächengebilde weist so eine verbesserte Hautverträglichkeit im Vergleich zu bekannten kohäsiven Binden auf Basis von Naturkautschuklatex auf. Gleichzeitig haftet das erfindungsgemäße Flächengebilde jedoch vorzugsweise auch nicht auf der Haut und den Haaren, wie haftklebende Pflaster und ist somit gut anzulegen und zu tragen, beispielsweise in Form einer Binde.

Dabei kann vorgesehen sein, dass die ersten diskreten Haftelemente mit weiteren Haftelementen auf der zweiten Seite zusammenwirken, die ebenfalls auf dem Substrat aufgebracht sind oder alternativ kann auch das Substratmaterial selber als Haftstruktur auf der zweiten Seite wirken. Hierbei können die Haftelemente auf der zweiten Seite aus einzelnen Fäden oder Fasern des textilen Substrates bestehen, wobei die einzelnen Fäden oder Fasern gleichzeitig die Haftmittel sind. Insbesondere ist die Vielzahl von ersten Haftelementen ausschließlich auf der ersten Seite des Flächengebildes aufgebracht. Es kann jedoch auch vorgesehen sein, eine weitere Vielzahl von ersten Haftelementen zusätzlich auf der zweiten Seite des Flächengebildes aufzubringen. Dies kann alternativ oder zusätzlich zur Vorsehung der Substratoberfläche als Haftstruktur erfolgen.

Gemäß einer besonders bevorzugten Ausführungsform kann vorgesehen sein, dass die ersten diskreten Haftelemente eine Flächendichte von 10² bis 10⁶ pro Quadratmeter auf dem Substrat im ungedehnten Zustand des Flächengebildes aufweisen.

Dabei können als erste Haftelemente flächige Formkörper definierter Länge L, Breite B und Höhe H verwendet werden, wobei auf mindestens einer ersten Oberfläche der Haftelemente Haftmittel vorgesehen sind. Das Verhältnis der Höhe H zur Länge dieser flächigen Formkörper liegt in einem Bereich von 1 bis 10⁻³. Derartige Haftelemente können in regulärer oder irregulärer diskreter Verteilung auf der Gesamtfläche des textilen Substrates, in der Form von Mustern, aber auch in Anhäufungen, Clustern, etc. vorgesehen sein.

Die flächigen Formkörper können vorzugsweise als Horizontalgeometrie (parallel zur ersten oder zweiten Seite des textilen Substrates) eine streifenförmige, quadratische, runde, halbrunde, ellipsenförmige, kreuz- oder sternförmige Basisfläche definierter Länge und Breite und eine lineare Vertikalgeometrie der Höhe H (senkrecht zur ersten oder zweiten Seite des textilen Substrates) aufweisen. Die Basisfläche kann auch eine dreieckige, viereckige, fünfeckige, sechseckige oder sonstige polygonale Form aufweisen, wobei Kanten und Winkel jeweils regulär oder irregulär sein können. Dabei können die Haftmittel auf nur einer Oberfläche des flächigen Formkörpers angebracht sein, diese können aber auch auf mehreren Oberflächen (Vorder-, Rückseite, Stirnseiten) in mehreren Achsenrichtungen orientiert angebracht sein.

Es kann beispielsweise vorgesehen sein, als Haftelemente Abschnitte einer Polymerfolie, auf welcher die Haftmittel vorgesehen sind, auf dem textilen Substrat anzubringen, wobei dies in definierter Verteilung und Flächendichte erfolgen kann. Die Flächendichte der Haftelemente kann dabei in einem Bereich von 10 ² bis 10 ⁶ Elementen je m² - bezogen auf den ungedehnten Zustand des Flächengebildes (Bestimmung gemäß EN 1773 - 1996) liegen. Die Haftmittel können auf einer oder mehreren Oberflächen (Vorder-, Rück- oder Stirnseite) der Abschnitte der Polymerfolie orientiert sein. Insbesondere können die ersten Haftelemente eine Grundfläche A mit einer Lange L und einer Breite B aufweisen, wobei auf der ersten Seite und auf der zweiten Seite der Haftelemente Haftmittel angebracht sind. Diese Haftmittel können Haken, Stengel, Pilzköpfe, Palmwedel, Lamellen sein, die mit der zweiten und der ersten Seite des textilen Substrates durch Verhaken eine Verbindung eingehen und dadurch eine wieder ablösbare Haftung eingehen.

Als Haftelemente können auch sogenannte Haftpartikel verwendet werden, deren Höhen-Längen-Verhältnis bevorzugt in der Größenordnung 1 bis 10⁻¹ liegt. Dabei werden partikuläre Formkörper mit kugelförmiger, ellipsoider, pyramidaler oder quaderförmiger Form verwendet. Die Oberflächen dieser Haftpartikel können allseitig mit Haftmitteln besetzt sein. Die Orientierung der Linearachse der Haftmittel ist dann vorzugsweise radial gerichtet, d.h. zum Schwerpunkt des Haftpartikels.

Die Anbringung der Haftelemente kann generell ein- oder beidseitig auf dem Substrat erfolgen.

Die auf der Oberfläche der Haftelemente angebrachten Haftmittel haben eine definierte Gestaltungsform, die so gewählt wird, dass eine haftende Verankerung der Haftelemente mit der zweiten Seite des Flächengebildes oder in eine Vielzahl zweiter Haftelemente erfolgt. Vorzugsweise haben die Haftmittel die Gestaltungsform von Stengeln mit oder ohne Kopfteil oder die Gestaltungsform von Haken. Beispiele sind den zuvor zitierten Druckschriften zu entnehmen. Die Gestaltungsform "Stengel" wird beispielsweise durch zylindrische, quaderförmige oder pyramidale, nadelförmige Formkörper repräsentiert, deren Längsachse einen definierten Winkel mit der Oberfläche des Haftelements bildet. Die Stengel können auch unter verschiedenen Winkeln, z.B. diagonal-gegenständig, d.h. alternierend eine Reihe unter einem Winkel von 70 ° zur Oberfläche des Haftelements, an der die jeweiligen Haftmittel festgelegt sind, die zweite Reihe unter einem Winkel von 110 ° zur vorgenannten Oberfläche, orientiert sein. Die Stengel können am Ende ein Kopfteil aufweisen, welches das leichtere Ineinandergreifen der Haftmittel ineinander sowie deren Verankerung, insbesondere zur Rückseite des Substrates, unterstützt. Es können Einfachköpfe und Mehrfachköpfe in Unterschiedlichen Formen, wie z.B. Scheiben, Pilzhüte, Flachteller verwendet werden, wobei deren Randlinienform glatt oder gelappt oder gezahnt sein kann.

Die Gestaltungsform "Haken" wird durch gebogene Formkörper in Hakenform mit einer gebogenen Spitze repräsentiert, welche analog zu den Geometrien, Winkeln und Anordnungen der beschriebenen Stengelformen auf der Oberfläche der Haftelemente platziert sind.

Die Haftmittel bestehen vorzugsweise aus dem gleichen Werkstoff wie die Haftelemente.

Für die Erfindung werden bevorzugt Haftelemente verwendet, bei denen die Haftmittel auf der Oberfläche in einer Flächendichte von 100 bis 5000 Stück je cm² angeordnet sind.

Sofern es sich bei den Haftelementen um Abschnitte einer Polymerfolie handelt, können starre Folien aus Standardkunststoffen wie Polyamide (PA), Polyester (PES), Polypropylen (PP) und Polyethylen (PE) oder auch weichflexible Polymerfolien aus Kunststoffen mit einer gewissen Dehnungselastizität wie metallocen-katalysiertes Polyolefin wie Polyethylen (PE) oder Polypropylen (PP) , thermoplastische Polyurethane (TPU) oder thermoplastische Kautschuke (TR) eingesetzt werden. Flexible Folien sind jedoch bevorzugt, da sie die elastische Verformungsfähigkeit des Substrats noch weniger beeinträchtigen.

Es kann auch vorgesehen sein, dass die Haftelemente verschiedene geometrische Formen aufweisen können. Auch können über das Flächengebilde verschiedene Flächendichten der Haftelemente vorgesehen sein.

Insbesondere können flächige, streifenförmige oder gitternetzförmige Zuschnitte derartiger Polymerfolien, die das Haftelement bilden und die Haftmittel tragen, verwendet werden. Es kann auch vorgesehen sein, dass die Haftelemente als Flächenelemente ausgebildet sind, bei denen dann die Haftmittel auf einer oder beiden Oberflächen des textilen Substrates angeordnet sind. Die Form der flächigen Zuschnitte kann rund, oval, quadratisch, rechteckig, polygonal, sternförmig sein oder auch Mischformen dieser Grundformen annehmen.

Die Haftelemente in Gestalt der Folienzuschnitte können durch geeignete Verbindungstechniken, wie z.B. Vernähen oder Verkleben oder Verschweißen oder Verpressen, auf der Oberfläche des Substrates befestigt werden. Vernähen meint die Befestigung der Haftelemente mit geeigneten Nähfäden und Nadeln, die das Substrat und die Haftelemente durchstechen, wobei die Verbindung durch den Nähfaden hergestellt wird. Verkleben meint die Befestigung der Haftelemente durch geeignete Klebstoffe in fester, flüssiger oder pastöser Form. Die Klebstoffe können auf Wasserbasis oder auf Lösemittelbasis aufgebaut sein oder es können thermoplastische Schmelzklebstoffe (Hotmelts) im geschmolzenen Zustand zum Einsatz kommen, indem die Klebstoffe durch Sprühen, Streichen, Rakeln, Pflatschen, Walzen, Gravurwalzen, Siebdrucksysteme oder Düsenauftragssysteme auf die glatte Rückseite der Haftelemente oder die Substratoberfläche aufgebracht werden, um die Haftelemente auf dem Substrat zu befestigen. Thermoplastische Schmelzklebstoffe können beispielsweise auch im kalten Zustand als Klebefilme, -fasern, -pulver, -vliese oder -netze zur Verwendung kommen, die auf der Rückseite der Haftelemente aufgebracht werden und nach Erwärmung im warmen, klebenden Zustand mit der Oberfläche des Substrates in klebende Verbindung gebracht werden. Verschweißen meint die direkte Verbindung der Haftelemente mit dem Substrat durch punktuelle oder vollflächige Erwärmung der Haftelemente mit einem Heißluft- oder Heißgasstrom oder einer offenen Flamme (Flammkaschierverfahren) oder mit beheizten Stempeln, Walzen, Kalandern oder Gravurzylindern oder Ultraschall-Sonotroden. Die Verbindung kommt durch die Erweichung oder Aufschmelzung der thermoplastischen Polymerfolie zustande. Es können alle geeigneten technischen Vorrichtungen und Verfahren zum Heißschweißen, Ultraschall-Schweißen oder Hochfrequenzschweißen verwendet werden, sofern die Haftmittel durch den Schweißvorgang nur unwesentlich zerstört werden. Verpressen meint, dass die Haftelemente selber auf der Oberfläche des Substrats befestigt werden, indem sich die Haftmittel durch den Pressdruck auf der Oberfläche des Substrats verankern. Die Anordnung kann dabei statistisch, ungeordnet oder in geometrisch gleichmäßiger Anordnung oder in Clustern, also so genannten Anhäufungen, erfolgen.

Hierbei ist wesentlich, dass die Dehnbarkeit des Flächengebildes 40 bis 300 % beträgt und die dehnungselastische Verformbarkeit des textilen Substrates möglichst wenig eingeschränkt wird.

Sofern die vorstehend beschriebenen partikelartigen Strukturen verwendet werden, auf denen die Haftmittel vorgesehen sind, bietet dies den besonderen Vorteil, dass hierdurch die Elastizität am wenigsten beeinträchtigt wird.

Der Anteil der Haftstruktur bezogen auf die einseitige Oberfläche des Grundtextils bzw. des textilen Substrates liegt in einem Bereich von 1 bis 50 %, vorzugsweise bei 1 bis 30 % und insbesondere vorzugsweise bei 5 bis 20 % der einseitigen Fläche des Substrats. Der Bezug hierbei ist die Fläche im ungedehnten Zustand des Textils, wobei die ungedehnte Fläche durch einfache Multiplikation aus der nach EN 1773 bestimmten Länge und Breite bestimmt wird.

Die geometrische Form, die Höhe und die Flächendichte der Haftmittel sowie der Aufbau des Textilsubstrats bestimmen die Größenordnung der zwischen den einzelnen Substratlagen wirkenden Haftkraft. Je nach Materialaufbau, Dichte, Elastizität und Flächengewicht des Grundtextils liegt die Haftkraft im Bereich zwischen 5 und 150 cN/cm. Bei leichten Grundtextilien für Fixierbinden (bis zu 50 g/m² gedehnt) genügt bereits eine Haftkraft von 5 bis 50 cN/cm für eine sichere Lagenhaftung. Bei schwererem Grundtextil beispielsweise für Stütz- und Kompressionsbinden (mit 50 bis 200 g/m² gedehnt) sind höhere Haftkräfte von 50 bis 150 cN/cm notwendig.

Sofern zur Bildung der Haftstruktur Folien als Haftelement eingesetzt werden, die auf das Substrat aufgebracht werden, wird eine Basisdicke von 50 bis 200 µm bevorzugt. Die hierauf angeordneten Haftmittel weisen eine Höhe von 100 bis 400 µm auf. Die Flächendichte der Haftmittel liegt dabei bevorzugt zwischen 100 und 5000 Stück pro Quadratzentimeter.

Gemäß einer besonders bevorzugten Ausführungsform kann vorgesehen sein, dass die ersten diskreten Haftelemente eine Flächendichte von 10² bis 10⁶ pro Quadratmeter auf dem Substrat im ungedehnten Zustand des Flächengebildes aufweisen, wobei die Haftkraft 50 bis 150 cN/cm beträgt. Ganz besonders bevorzugt kann vorgesehen sein, dass die ersten diskreten Haftelemente eine Flächendichte von 10² bis 10⁶ pro Quadratmeter auf dem Substrat im ungedehnten Zustand des Flächengebildes aufweisen, wobei der Anteil der Haftstruktur bezogen auf die einseitige Oberfläche des Grundtextils bzw. des textilen Substrates in einem Bereich von 1 bis 50 % liegt und die Haftkraft 50 bis 150 cN/cm beträgt.

Des Weiteren ist vorgesehen, dass das textile Substrat mindestens eine Kettfadenschar aufweist, die aus dehnungselastischen Textilfäden, beispielsweise aus hochgedrehten Baumwollfäden, Elastodien, Elastan, thermoplastischem Kautschuk oder texturierten Multifilamentgarnen aus Chemiefasern, bestehen. Derartige dehnungselastische Kettfäden können teilweise auch in Kombination mit anderen unelastischen oder starren Kettfäden oder geeigneten Schussfäden oder Vliesstoffen verarbeitet werden.

Bevorzugt sind medizinische Flächengebilde, die ein textiles Substrat umfassen, das eine erste und mindestens eine zweite Kettfadenschar umfasst. Das medizinische Flächengebilde gemäß Anspruch 1 umfaßt eine erste und mindestens eine zweite Kettfadenschar, wobei die erste Kettfadenschar aus elastischen Fäden besteht und die mindestens zweite Kettfadenschar aus unelastischen Fäden besteht.

Darüber hinaus können die medizinischen Flächengebilde ein Substrat umfassen, das weiterhin einen oder mehrere Schussfäden umfasst. Insbesondere kann das medizinische Flächengebilde ein Substrat umfassen, das eine erste und eine zweite Kettfadenschar und einen Schussfaden umfasst. Dabei ist die erste Kettfadenschar dehnungselastisch ausgestattet wogegen die zweite Kettfadenschar sowie der Schussfaden aus unelastischen Fäden bestehen.

Es kann jedoch auch vorgesehen sein, das das textile Substrat ein Nonwoven umfasst. Ganz besonders bevorzugt sind medizinische Flächengebilde, die ein textiles Substrat umfassen, das ein Gewebe, Gewirke, Gestrick, Nähvlies oder Nähgewirk ist. Falls das textile Substrat ein Nähgewirk ist, insbesondere ein Malimo-Nähgewirk, kann vorgesehen sein, dass dieses Gewirk zusätzlich durch ein Nonwoven in Form eines Spinnvliesstoffes oder eines Stapelfaser-Vliesstoffes verstärkt wird. Insbesondere weisen derartige medizinische Flächengebilde mit einem Gewebe, Gewirke, Gestrick, Nähvlies oder Nähgewirk als textiles Substrat eine Flächengewicht von mindestens 10 g/ m² und höchstens 350 g/ m² besonders bevorzugt von mindestens 20 g/ m² und höchstens 60 g/ m² oder mindestens 70 g/ m² und höchstens 350 g/ m² auf.

Die Haftkraft zwischen den Lagen des erfindungsgemäßen medizinischen Flächengebildes wird als Abschälfestigkeit des Flächengebildes nach DIN EN 12242 bestimmt und beträgt mindestens 5 cN/cm, um eine ausreichende Lagenhaftung zu gewährleisten. Insbesondere soll das medizinische Flächengebilde eine Haftkraft von 5 - 120 cN/cm und ganz besonders bevorzugt 20 - 120 cN/cm aufweisen.

Derartige medizinische Flächengebilde können insbesondere als elastische Binden für Fixier-, Stütz-, Entlastungs- und Kompressionsanwendungen am menschlichen oder tierischen Körper eingesetzt werden.

Mit einem entsprechenden Flächengebilde kann ein elastisches Verbandmaterial bzw. eine elastische Binde mit einer haftenden Oberfläche realisiert werden, ohne dass dazu Klebemittel kohäsiver oder adhäsiver Natur an der Oberfläche verwendet werden müssen. Der Hafteffekt resultiert rein mechanisch aus einem Ineinandergreifen der Haftstrukturen bzw. der Haftmittel in Verbindung mit dem textilen Substrat. Die Verbindungen, die aus dem Ineinandergreifen der Haftmittel resultierten, sind wieder lösbar, ohne dass eine wesentliche Schädigung des textilen Substrates erfolgt. Darüber hinaus werden auf vorstehend beschriebene Weise Haftbinden zugänglich, die eine verbesserte Alterungs-, Lager- und Lichtbeständigkeit erreichen, da die verwendeten Haftmittel, vorzugsweise Polymerfolien mit aufgebrachten Haftmitteln, deutlich bessere Alterungs-, Lager- und Lichtstabilität aufweisen als die bekannten kohäsiven oder adhäsiven Klebemittel. Ein weiterer Vorteil ist, dass an ein am Körperteil befestigtes, erfindungsgemäßes Flächengebilde, welches die Haftelemente auf der Außenseite (der dem Körper abgewandten Seite) trägt, weitere nicht klebende Textilprodukte angeheftet werden können, wie beispielsweise weitere Binden, Polstermittel, Kompressen, Hilfsmittel etc., für den Fall, dass diese eine Verbindung mit den Haftelementen eingehen. Dies ist beispielsweise vorteilhaft bei sogenannten Mehrlagenverbänden (Multi-Layerverbänden), die bevorzugt in der Kompressionstherapie zur Behandlung von venösen Beinleiden verwendet werden. Hierbei wird eine erste Binde direkt am Unterschenkel angewickelt und danach eine, zwei oder drei weitere Binden unterschiedlicher Qualität schichtweise übereinander gewickelt, wobei die einzelnen Binden keinerlei Haftung oder Verklebung miteinander eingehen. Der resultierende, mehrlagige Kompressionsverband soll dann in der Regel über mehrere Wochen als Dauerverband vom Patienten getragen werden. Eine erfindungsgemäße Haftbinde wie oben beschrieben stellt in diesem Mehrlagenverband eine innige haftende Verbindung zu der jeweils nächsten Bindenschicht her und bringt Vorteile in Richtung Rutschfestigkeit und sichere Platzierung der einzelnen Verbandlagen aufeinander.

Bei den erfindungsgemäßen Flächengebilden wurde die Dehnbarkeit gemäß der DIN 61632 ebenso wie die Rückzugsfähigkeit bestimmt. Zur Ermittlung der Luftdurchlässigkeit wurde die DIN 53887 herangezogen.

Im Folgenden sollen verschiedene Haftelemente sowie ein Flächengebilde zur Verdeutlichung anhand einer Zeichnung erläutert werden. Dabei zeigen:
- Figuren 1 - 5:: verschiedene Ausgestaltungen der Haftelemente
und
- Figur 6:: einen Teil eines erfindungsgemäßen Flächengebildes in einer Draufsicht.

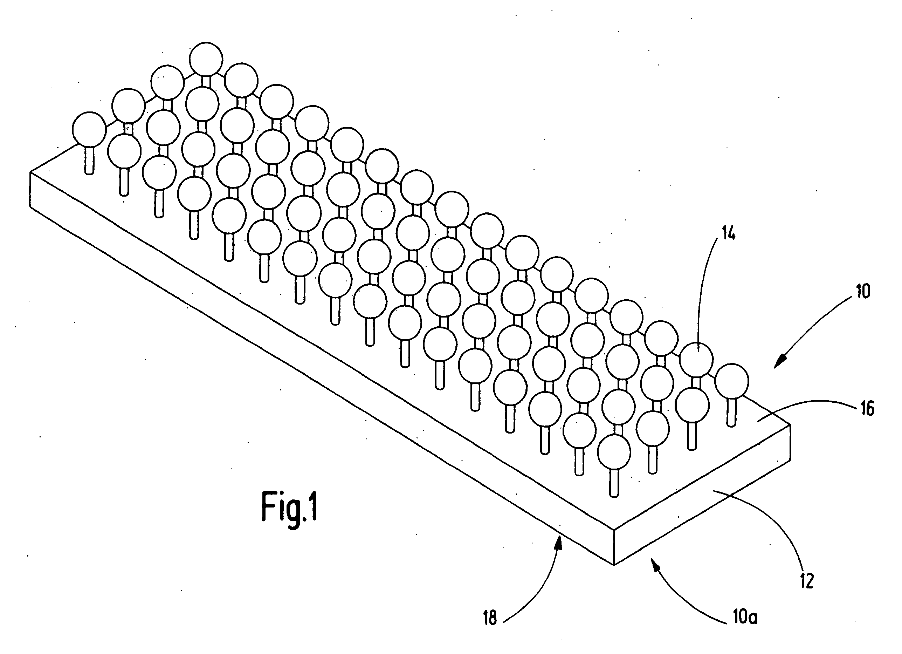

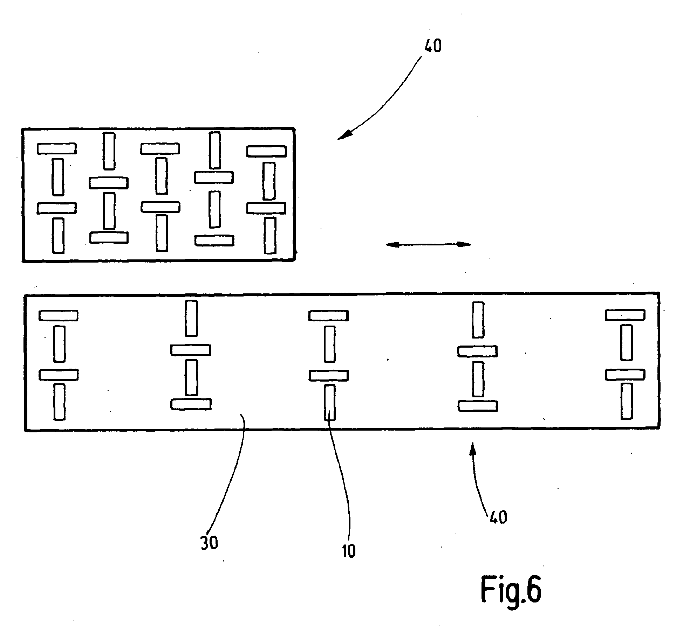

Figur 1 zeigt ein erstes Haftelement 10a, das als flächiger Formkörper 10 ausgebildet ist und bei dem auf einer Polymerfolie 12 mit einer Dicke von 100 µm Haftmittel 14 in Form von Stengeln mit Pilzköpfen vorgesehen sind, wobei die Pilzköpfe kalottenförmig ausgebildet sind. Die Haftmittel 14 sind hierbei auf lediglich einer Seite 16 der Folie aufgebracht. Mit der zweiten Seite 18 wird die Folie dann an ein textiles Substrat (nicht dargestellt) zur Bildung eines erfindungsgemäßen Flächengebildes 40 (Figur 6) angehaftet. Dies kann über ein Schweißverfahren erfolgen. Derartige Haftelemente 10a können ein- oder beidseitig auf dem Substrat vorgesehen sein.

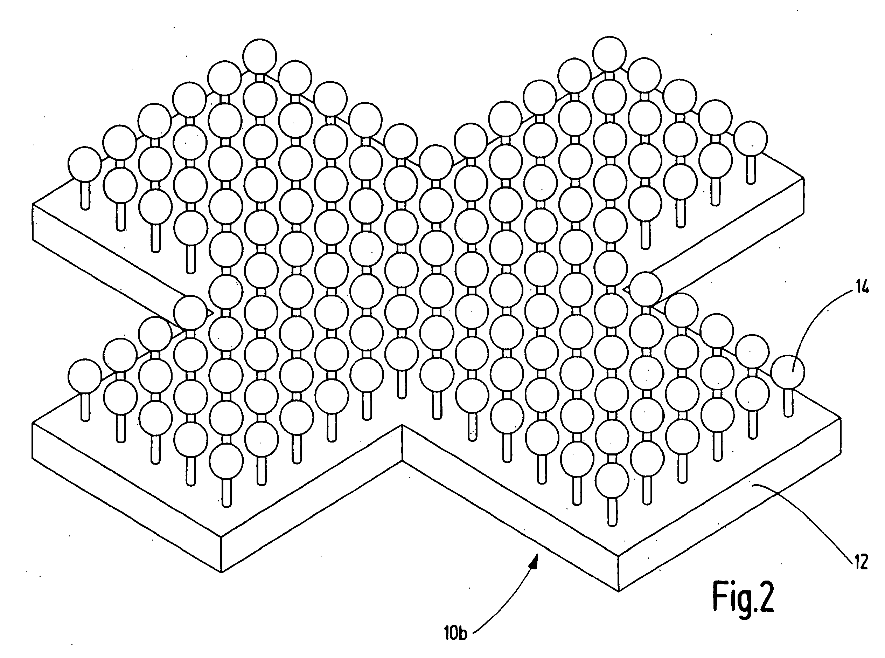

Figur 2 zeigt eine analoge Gestaltung eines Haftelements 10b, wobei hier die Grundfläche der Polymerfolie nicht rechteckig wie in Figur 1 sondern als eine kreuzförmige Basisfläche vorgesehen ist. Die Vorsehung und Anordnung der Haftmittel 14 erfolgt gemäß Figur 1.

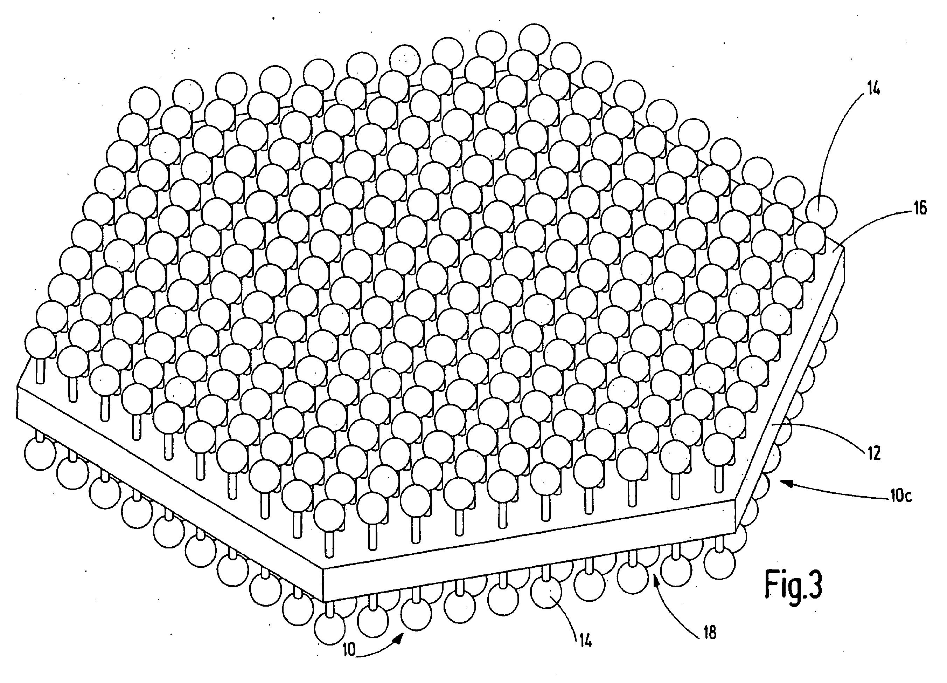

Figur 3 zeigt nun ein Haftelement 10c in Form eines flächigen Formkörpers 10 mit polygonaler, nämlich sechseckiger Grundfläche. Anders als bei den vorangehenden Beispielen sind hier Haftmittel 14 auf beiden Oberflächen 16, 18 der Polymerfolie 12 angebracht. Die Haftmittel 14 auf der einen Seite 16 dienen dann zum Verbinden mit einer ersten Seite eines textilen Substrats. D.h. die Anhaftung der Haftelemente 10c am Substrat erfolgt mittels der Haftmittel 14 und die Haftmittel 14 der anderen Seite 18 dienen dann zum Verbinden der ersten Seite des Substrats mit einer Haftstruktur auf einer zweiten Seite des Substrats.

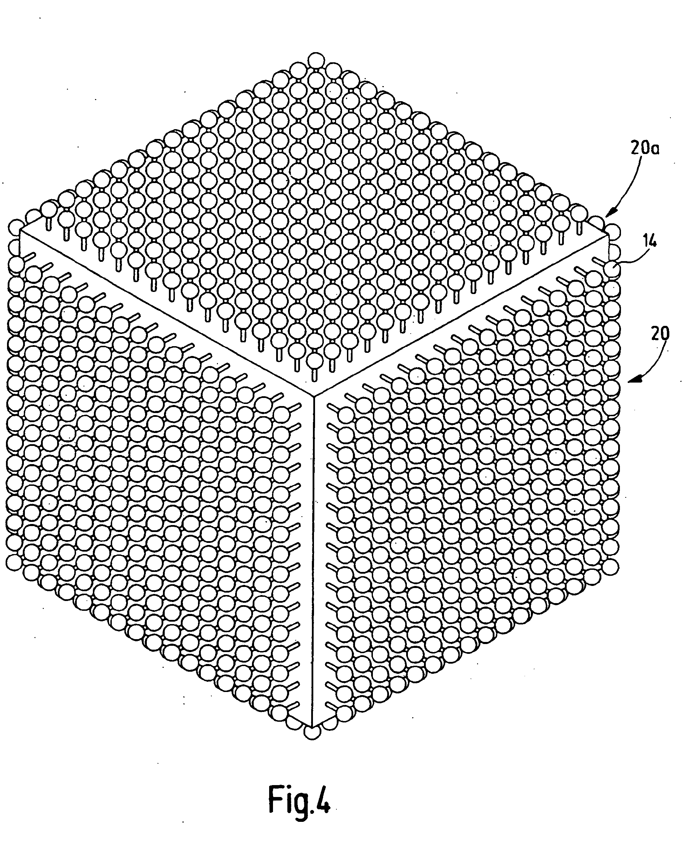

Figur 4 zeigt schließlich einen sogenannten Haftpartikel 20a, der durch einen dreidimensionalen Formkörper 20 gebildet ist und der eine würfelförmige Geometrie aufweist. Der Partikel 20a trägt dabei Haftmittel 14, die den vorstehend beschriebenen entsprechen. Die Haftmittel 14 sind dabei allseitig angebracht und dienen sowohl zur Verbindung der ersten Substrat- bzw. Flächengebildeseite mit einer zweiten Substrat- bzw. Flächengebildeseite als auch zur Befestigung des Haftpartikels 20a am Substrat.

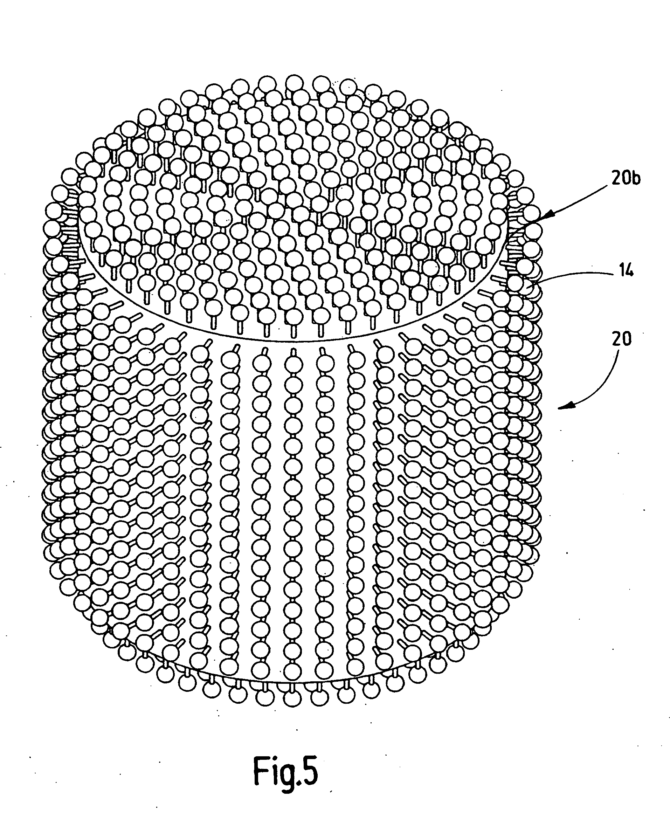

Schließlich zeigt Figur 5 eine analoge Gestaltung zu Figur 4, wobei hier der Haftpartikel 20b jedoch eine zylindrische Form aufweist.

Die Erfindung soll im Folgenden darüber hinaus anhand zweier Beispiele beschrieben werden.

### Beispiel 1:

### Haftbinde auf Basis eines elastischen Mullbindengewebes für Fixierzwecke:

Als Trägertextil (Substrat 30) für eine erfindungsgemäße Haftbinde wurde ein elastisches Mullbindengewebe in definierter Breite, z.B. 8 cm, nach DIN 61634 mit folgendem Aufbau verwendet:

| Materialaufbau Gewebe | 71 % Viskose, 29 % Polyamid, Leinwandbindung |
|---|---|
| Kettfaden 1 / Kettfaden 2 | 17 tex Viskose / 78dtex f 17 x 2 Polyamid texturiert |
| Fadenzahlen Kettfaden 1 / Kettfaden 2 | 56 / 56 pro 10 cm Breite |
| Schussfaden | 17 tex Viskose Stapelfasergarn |
| Schussfadendichte | 36 Doppelschuss je 10 cm gedehnt (DIN 61632) |
| Flächengewicht gedehnt | 32 g/ m² (DIN 61632) |
| Elastizität | In Längsrichtung (Kettrichtung) |
| Dehnbarkeit / Rückzug | 140 % / 99 % (DIN 61632) |
| Luftdurchlässigkeit | 6000 l / m² sec (DIN 53887) |

Zur Herstellung der erfindungsgemäßen Haftbinde wird die unter dem Handelsnamen MICROPLAST 65445 der G. Binder GmbH & Co., Holzgerlingen, Deutschland, kommerziell erhältliche Klettverschluss-Polymerfolie als Haftelement 10a verwendet, welche die Haftmittel 14 in Gestalt von Stengeln mit Pilzköpfen auf der Oberfläche enthält. Nachstehend die technischen Daten dieser Haftelement-Folie:

| Material | Polypropylen extrudiert, transparent |
|---|---|
| Flächengewicht | 135 g/m² |
| Dicke gesamt | 360 µm |
| Aufbau | Haftmittel einseitig, Rückseite glatt |
| Haftmittel | Stengel mit Pilzkopf hexagonal, Höhe 250 µm |
| Flächendichte der Haftmittel | 288 Stück je cm² |
| Anordnung | Reguläre Verteilung (Schachbrettmuster) |

Die Folie wird in Rollenform definierter Breite, z.B. 40 cm, einem Quetschmesseraggregat zugeführt und in Streifen der Breite 3 mm geschnitten, welche dann im Anschluss wiederum in Querrichtung in Zuschnitte der Länge 15 mm geschnitten, gestanzt oder mit geeigneten Verfahren zerkleinert werden. Jeder in dieser Weise durch Zuschnitt gewonnene Folienabschnitt 12 weist eine rechteckige Fläche von 0,45 cm² auf. Die Zuschnitte werden in einem regelmäßig wiederkehrenden Musterrapport nach Art eines orthogonalen Kreuzgittermusters (siehe Figur 6) auf einen Streifen des Substrats 30 einseitig wie folgt auflaminiert:

Die Zuschnitte werden auf ihrer glatten Rückseite mit einer für eine permanente Verankerung auf dem Trägersubstrat 30 ausreichenden Menge Haftkleber (z.B. Acrylat-Dispersionskleber, Schmelzkleber, Lösemittelhaltiger Kautschukkleber wie z.B. PATTEX, Henkel KGaA, Deutschland) durch Sprühen, Tauchen, Rakeln Streichen etc. beschichtet und dann anschließen gemäß der Musterung in Figur 6, Darstellung a) auf der Oberfläche des spannungslos vorgelegten Substrats 30 so angeordnet, dass die Schwerpunkte der Zuschnitte jeweils einen Abstand von 3 cm aufweisen bzw. ein regelmäßiges Gitter mit der Gitterkonstanten 3 cm bilden. Nach Anpressen und Trocknen bzw. Abkühlen der Klebefugen haften die Zuschnitte fest mit ihrer Rückseite auf dem Substrat 30 und die Haftmittel 14 (Pilzköpfe) zeigen nach außen. Im ungedehnten Zustand des Flächengebildes 40 beträgt der flächenbezogene Anteil der Folienzuschnitte auf dem Substrat 30 ca. 7,5 %, bezogen auf den gedehnten Zustand ca. 3 %. Der gedehnte Zustand in Pfeilrichtung ist in Figur 6, Darstellung b) gezeigt.

Das in dieser Weise hergestellte Textilband verfügt über die gesamte Lauflänge über eine Haftoberfläche mit partieller und diskreter Beschichtung mit Haftelementen. Die Flächendichte der Haftelemente 10a auf der einen Seite des Textilsubstrates liegt bei 1,1· 10³ Stück je Quadratmeter ungedehnt.

Beim Verpressen der haftenden Seite mit sich selbst sowie mit der unbeschichteten Rückseite tritt eine Lagenhaftung nach Art des Kletteffekts ein, wobei sich die Pilzköpfe der Haftfolien-Zuschnitte mit einzelnen Fasern bzw. Filamenten der Kett- und Schussfäden des textilen Substrats 30 verhaken. Zur Trennung der Lagen ist eine definierte Kraft erforderlich. Bei Anwendung des "Peel-Test-Prüfverfahrens (Schälfestigkeit)" gemäß DIN EN 12242-1999 ergibt sich die Haftkraft zu 10 cN/cm, d.h. zur Trennung des Lagenverbundes bei einer 10 cm breiten Binde sind 100 cN notwendig.

Das in dieser Weise hergestellte Textilband mit einseitiger Haftoberfläche hat folgende Eigenschaften:
Flächengewicht gedehnt: 33,5 g/m²
Dehnbarkeit DIN 61632: 120 %
Rückzug DIN 61632: 98 %
Luftdurchlässigkeit DIN 53887: 5600 l/m² sec

Vor der Anwendung wird das Textilband im weitgehend entspannten (ungedehnten) Zustand analog zu handelsüblichen Fixierbinden zu einer gewickelten Binde in Rollenform gewickelt, wobei die Seite mit den Haftelementen auf der Außen - oder Innenseite, vorzugsweise auf der Außenseite, der gewickelten Binde angeordnet ist. Im letzteren Fall befinden sich die Haftelemente dann hautseitig, während die Außenfläche des Verbandes die unveränderte Rückseite des Basistextils darstellt. Die Haftbinde gemäß Beispiel 1 kann beispielsweise zur Fixierung einer Wundkompresse im Wundbereich an einer verletzten Körperstelle verwendet werden. Dazu wird die Wundkompresse auf die Wunde aufgelegt und dann durch Abrollen der Haftbinde unter leichter Spannung das verletzte Körperteil einschließlich der Wundkompresse umwickelt, wobei sich die Haftelemente mit der Bindenoberfläche sowie gegebenenfalls auch mit der Rückseite der Wundkompresse verhaken. Dadurch entsteht ein haftender, rutschsicherer Fixierverband, der die Wundkompresse in der gewünschten Position hält.

### Beispiel 2:

### Haftbinde auf Basis eines elastischen Nonwovens als Stütz-/Kompressionsbinde:

Als Trägertextil (Substrat 30) für eine erfindungsgemäße Haftbinde wurde eine elastische Non Woven-Konstruktion verwendet, die durch Übernähen eines starren Polyester-Vliesstoffes mit dauerelastischen Elastanfäden in Längsrichtung unter Anwendung der Nähwirktechnik MALIWATT erhalten wurde. Einzelheiten dieses Trägertextils siehe nachstehende Tabelle

| Materialaufbau Elastisches Nonwoven | 80 % Polyester, 20 % Polyurethan - Elastan |
|---|---|
| Grund - Vlies | PES-Stapelfaservlies, 25 g/m², thermisch verfestigt, geprägt |
| Nähfaden | 133 dtex Elastan (DORLASTAN, BAYER) |
| Nähfadendichte | 45 Fäden je 10 cm Breite |
| Nähfaden- Stichlänge, Bindung | 3 mm, offene Franse |
| Flächengewicht gedehnt | 32 g/ m² |
| Elastizität | In Längsrichtung (Kettrichtung) |
| Dehnbarkeit / Rückzug nach DIN 61632 | 250 % / 99 % |
| Vliesbreite | 100 cm |
| Luftdurchlässigkeit DIN 53887 | 2000 l / m² sec ungedehnt |

Zur Herstellung der erfindungsgemäßen Haftbinde wird die unter dem Handelsnamen MICROPLAST 65445 der G. Binder GmbH & Co., Holzgerlingen, Deutschland, kommerziell erhältliche Klettverschluss-Polymerfolie als Haftelement 10a verwendet, welche die Haftmittel 14 in Gestalt von Stengeln mit Pilzköpfen auf der Oberfläche enthält. Nachstehend die technischen Daten dieser Haftelement-Folie:

| Material | Polypropylen extrudiert, transparent |
|---|---|
| Flächengewicht | 135 g/m² |
| Dicke gesamt | 360 µm |
| Aufbau | Haftmittel einseitig, Rückseite glatt |
| Haftmittel | Stengel mit Pilzkopf hexagonal, Höhe 250 µm |
| Flächendichte der Haftmittel | 288 Stück je cm² |
| Anordnung | Reguläre Verteilung (Schachbrettmuster) |

Die Folie wird in Rollenform mit 100 cm Breite einem Quetschmesseraggregat zugeführt und in Querrichtung in Streifen der Breite 3 mm geschnitten, welche dann im Anschluss mit einer Greifervorrichtung im Abstand von 50 mm in wiederholendem Rapport mit der glatten Seite auf die Oberfläche der zugeführten Nonwoven Bahn aufgelegt werden. Jeder in dieser Weise durch Zuschnitt gewonnene Folienabschnitt weist eine rechteckige Fläche von 30 cm² Fläche auf. Die Zuschnitte werden mit mehreren Ultraschall-Schweißköpfen (Hersteller: SONOTRONIC, Karlsbad) punktuell oder vollflächig mit dem Substrat 30 über die gesamte Breite durch die beim Anpressen der Sonotroden gebildete Reibungswärme thermisch verbunden, wobei sich die glatte Seite der Haftelemente fest mit der Textiloberfläche des Substrats verbindet. Die Haftmittel 14 (Pilzköpfe) zeigen nach außen. Im ungedehnten Zustand beträgt der flächenbezogene Anteil der Haftfolienzuschnitte auf dem Textilgrund des Substrats 30 a. 6 %, bezogen auf den gedehnten Zustand ca. 1,7 %.

Die in dieser Weise hergestellte Breittextilbahn wird mit Schneidmessern (vorzugsweise Heißschneiden oder Ultraschallschneiden) in Bindenstreifen definierter Breite, z.B. 6, 8, 10, 12 cm geschnitten, welche dann ebenso wie die Breitextilbahn über die gesamte Lauflänge über eine Haftoberfläche mit partieller Beschichtung mit Haftmitteln verfügen. Die Flächendichte der Haftelemente auf der einen Seite des Textilsubstrates 30 liegt für die 10 cm Bindenbreite bei 2 · 10² Stück je Quadratmeter ungedehnt bzw. für die 6 cm Bindenbreite bei 3,3 · 10² Stück je Quadratmeter ungedehnt.
Beim Verpressen der haftenden Seite mit sich selbst sowie mit der unbeschichteten Rückseite tritt eine Lagenhaftung nach Art des Kletteffekts ein, wobei sich die Pilzköpfe der Haftfolien-Zuschnitte mit einzelnen Fasern bzw. Filamenten der Fasern des Nonwovens verhaken. Zur Trennung der Lagen ist eine definierte Kraft erforderlich. Bei Anwendung des "Peel-Test-Prüfverfahrens (Schälfestigkeit)" gemäß DIN EN 12242 ergibt sich die Haftkraft zu 35 cN/cm, d.h. zur Trennung des Lagenverbundes bei einer 10 cm breiten Binde sind 350 cN notwendig. Damit ist eine ausreichend feste bzw. sichere Verbindung der Lagen im fertigen Verband sichergestellt.

Das in dieser Weise hergestellte Flächengebilde 40 mit einseitiger Haftoberfläche hat folgende Eigenschaften:
Flächengewicht gedehnt: 40 g/m²
Dehnbarkeit DIN 61632: 230 %
Rückzug DIN 61632: 99 %
Luftdurchlässigkeit DIN 53887: 1800 l/m² sec

Zur Anwendung wird das Flächengebilde 40 wiederum im entspannten Zustand zur Binde gewickelt, wobei die Haftelemente 10a entweder außen- oder innen liegend orientiert sein können.

In analoger Weise kann das Nonwoven auch auf der Vorder- und Rückseite, d.h. beidseitig, mit streifenförmigen Zuschnitten der Haftfolie laminiert werden. Im ungedehnten Zustand beträgt der flächenbezogene Anteil der Haftfolienzuschnitte auf dem Textilgrund des Substrats dann ca. 12 %, bezogen auf den gedehnten Zustand ca. 3,5 %. Das Flächengewicht erhöht sich auf ca. 48 g/m². Auf einen Verband aus diesem beidseitig mit Haftelementen ausgestatteten Substrat können weitere Bindenlagen haftend fixiert werden, da diese eine Verbindung mit den außen liegenden Haftelementen eingehen. Eine Haftbinde gemäß Beispiel 2 kann beispielsweise zur Kompressionsbehandlung von venösen Beinerkrankungen angewendet werden. Dazu wird die Haftbinde unter leichtem, gleichmäßigem Verzug abgerollt und am Zehengrundgelenk beginnend in definierter Lagenzahl, z. B. dreilagig, der gesamte Unterschenkel des Patienten am erkrankten Bein umwickelt. Die Haftelemente verhaken dabei mit der rückseitigen Bindenoberfläche, wodurch ein haftender, rutschsicherer Kompressionsverband entsteht, der im Rahmen einer Entstauungs- und Kompressionstherapie über mehrere Tage getragen wird. Auf den außen liegenden Haftelementen können weitere Verbandmaterialien, z. B. Mullbinden, Polsterbinden beim Umwickeln fixiert werden.

## Patentansprüche

1. Medizinisches haftfähiges Flächengebilde (40), das dehnungselastisch ausgebildet ist und eine Dehnbarkeit zwischen 40 % und 300 % aufweist, welches ein textiles Substrat (30)umfasst, wobei das Substrat (30) eine erste und eine zweite Seite besitzt und mindestens eine Kettfadenschar umfasst, wobei auf der ersten Seite des Substrats (30)eine erste mechanische Haftstruktur aufgebracht ist, die aus einer Vielzahl diskreter erster Haftelemente (10, 20) besteht und die erste mechanische Haftstruktur mit einer zweiten mechanischen Haftstruktur auf der der ersten Seite gegenüberliegenden zweiten Seite des Substrats (30) in haftende Verbindung bringbar ist, wobei das Substrat eine erste und mindestens eine zweite Kettfadenschar umfasst, wobei die erste Kettfadenschar aus elastischen Fäden besteht und die mindestens zweite Kettfadenschar aus unelastischen Fäden besteht.

2. Medizinisches Flächengebilde nach Anspruch 1, wobei das Flächengebilde (40) eine Haftkraft von 5 - 120 cN/cm aufweist und der Flächenanteil der Haftstruktur bezogen auf die einseitige Oberfläche des ungedehnten textilen Substrates (30) in einem Bereich von 1 bis 50% der einseitigen Fläche des Substrats (30) liegt.

3. Medizinisches Flächengebilde nach Anspruch 1, wobei das Flächengebilde (40) eine Haftkraft von 5 - 120 cN/cm aufweist und die ersten Haftelemente (10, 20) in diskreter Verteilung eine Flächendichte von 10² bis 10⁶ Stück pro Quadratmeter auf dem ungedehnten Flächengebilde aufweisen.

4. Medizinisches Flächengebilde nach Anspruch 1, wobei das Flächengebilde (40) eine Haftkraft von 5 - 120 cN/cm aufweist und die ersten Haftelemente (10, 20) in diskreter Verteilung eine Flächendichte von 10² bis 10⁶ Stück pro Quadratmeter auf dem ungedehnten Flächengebilde aufweisen und der Flächenteil der Haftstruktur bezogen auf die einseitige Oberfläche des ungedehnten textilen Substrates (30) in einem Bereich von 1 bis 50% der einseitigen Fläche des Substrates (30) liegt.

5. Medizinisches Flächengebilde nach einem der vorangehenden Ansprüche, wobei das Substrat (30) einen oder mehrere Schussfäden umfasst.

6. Medizinisches Flächengebilde nach einem der vorangehenden Ansprüche, wobei das Substrat (30) ein Nonwoven umfasst.

7. Medizinisches Flächengebilde nach einem der vorangehenden Ansprüche, wobei das Flächengebilde (40) eine Länge L und eine Breite B aufweist, wobei das Flächengebilde über die gesamte Länge L homogen dehnungselastisch ist.

8. Medizinisches Flächengebilde nach einem der vorangehenden Ansprüche, wobei die ersten Haftelemente (10, 20) durch flächige Formkörper (10) mit einem Höhen-Längen-Verhältnis von 1 bis 10⁻³ oder Haftpartikel (20) mit einem Höhen-Längen-Verhältnis von 1 bis 10⁻¹ gebildet sind und auf den Haftelementen (10, 20) Haftmittel (14) vorgesehen sind.

9. Medizinisches Flächengebilde nach einem der vorangehenden Ansprüche, wobei die Haftmittel (14) eine Stengel- oder Hakenform aufweisen.

10. Medizinisches Flächengebilde nach einem der vorangehenden Ansprüche, wobei die zweite mechanische Haftstruktur durch das Substrat (30) selbst gebildet ist.

11. Medizinisches Flächengebilde nach einem der vorangehenden Ansprüche, wobei das textile Substrat (30) ein Gewebe, Gewirke, Gestrick, Nähgewirk oder ein Nähvlies ist.

12. Medizinisches Flächengebilde nach einem der vorangehenden Ansprüche, wobei das Flächengebilde (40) eine Flächengewicht von mindestens 10 g/ m² und höchstens 350 g/ m² aufweist.

13. Medizinisches Flächengebilde nach einem der vorangehenden Ansprüche, wobei es eine elastische Kompressionsbinde oder -bandage oder eine elastische Fixierbinde oder - bandage oder eine elastische Stützbinde oder -bandage ist.

14. Verfahren zur Herstellung eines medizinischen haftfähigen Flächengebildes nach einem der Ansprüche 1 bis 13, wobei die mit Haftmitteln besetzten Haftelemente in einem ersten Schritt durch Vereinzeln von Polymerfolien in Gestalt von Zuschnitten der Länge L, Breite B und Höhe H hergestellt werden und die Zuschnitte in einem zweiten Schritt auf eine Oberfläche eines Textilsubstrats in diskreter Verteilung angeordnet werden und dabei durch Vernähen oder Verkleben oder Verschweißen oder Verpressen fest mit dem Substrat verbunden werden.

15. Verfahren zur Herstellung eines medizinischen haftfähigen Flächengebildes nach einem der Ansprüche 1 bis 13, wobei mit Haftmitteln besetzte Haftpartikel direkt durch Extrusion in definierter Länge L, Breite B und Höhe H hergestellt werden und dann auf einer Oberfläche eines Textilsubstrats in diskreter Verteilung angeordnet werden und dabei durch Vernähen oder Verkleben oder Verschweißen oder Verpressen fest mit dem Substrat verbunden werden.

## Claims

1. Medical adhesive planar structure (40), which is designed to be elastically extensible and provides an extensibility between 40% and 300% and which comprises a textile substrate (30), wherein the substrate (30) has a first and a second side and comprises at least one warp thread group, wherein, on the first side of the substrate (30), a first mechanical adhesive structure is applied, which consists of a multiplicity of discrete first adhesive elements (10, 20), and the first mechanical adhesive structure can be brought into an adhesive connection with a second mechanical adhesive structure on the second side of the substrate (30) disposed opposite to the first side, wherein the substrate comprises a first and at least a second warp thread group, wherein the first warp thread group consists of elastic threads and the at least second warp thread group consists of non-elastic threads.

2. Medical planar structure according to claim 1, wherein the planar structure (40) provides an adhesive force from 5 - 120 cN/cm and the proportion by area of the adhesive structure relative to the surface on one side of the un-expanded textile substrate (30) is disposed within a range from 1 to 50% of the area of one side of the substrate (30).

3. Medical planar structure according to claim 1, wherein the planar structure (40) provides an adhesive force from 5 - 120 cN/cm and the first adhesive elements (10, 20) provide in a discrete distribution a density per unit area from 10² to 10⁶ items per square metre on the un-expanded planar structure.

4. Medical planar structure according to claim 1, wherein the planar structure (40) provides an adhesive force from 5 - 120 cN/cm, and the first adhesive elements (10, 20) provide in a discrete distribution a density per unit area from 10² to 10⁶ items per square metre on the un-expanded planar structure, and the proportion by area of the adhesive structure relative to the surface on one side of the un-expanded textile substrate (30) is disposed within a range from 1 to 50% of the area of one side of the substrate (30).

5. Medical planar structure according to any one of the preceding claims, wherein the substrate (30) comprises one or more weft threads.

6. Medical planar structure according to any one of the preceding claims, wherein the substrate (30) comprises a non-woven fabric.

7. Medical planar structure according to any one of the preceding claims, wherein the planar structure (40) provides a length L and a width B, wherein the planar structure is elastically extensible in a homogeneous manner over the entire length L.

8. Medical planar structure according to any one of the preceding claims, wherein the first adhesive elements (10, 20) are formed by planar formed elements (10) with a height-to-length ratio from 1 to 10⁻³ or adhesive particles (20) with a height-to-length ratio from 1 to 10⁻¹, and adhesive means (14) are provided on the adhesive elements (10, 20).

9. Medical planar structure according to any one of the preceding claims, wherein the adhesive means (14) provide a stalk shape or a hook shape.

10. Medical planar structure according to any one of the preceding claims, wherein the second mechanical adhesive structure is formed by the substrate (30) itself.

11. Medical planar structure according to any one of the preceding claims, wherein the textile substrate (30) is a woven, meshed, knitted, stitch-bonded or stitched fleece fabric.

12. Medical planar structure according to any one of the preceding claims, wherein the planar structure (40) provides a weight by area of at least 10 g/m² and at most 350 g/m².

13. Medical planar structure according to any one of the preceding claims, wherein it is an elastic compression dressing or bandage or an elastic fixation dressing or bandage or an elastic support dressing or bandage.

14. Method for the manufacture of a medical adhesive planar structure according to any one of claims 1 to 13, wherein, in a first step, the adhesive elements provided with adhesive means are manufactured through the separation of polymer films in the form of cut pieces of the length L, width B and height H, and in a second step, the cut pieces are arranged in discrete distribution on a surface of a textile substrate and, in this context, are firmly connected to the substrate by sewing or gluing or welding or compression.

15. Method for the manufacture of a medical adhesive planar structure according to any one of claims 1 to 13, wherein adhesive particles provided with adhesive means are manufactured directly in a defined length L, width B and height H by extrusion, and are then arranged in discrete distribution on a surface of a textile substrate and, in this context, firmly connected to the substrate by sewing or gluing or welding or compression.

## Revendications

1. Produit en nappe (40) adhésif à usage médical, réalisé avec une élasticité d'allongement et possédant une extensibilité comprise entre 40 % et 300 %, comprenant un support textile (30), qui possède une première face et une seconde face et comportant au moins un système de fils de chaîne, une première structure adhésive mécanique étant appliquée sur la première face du support (30), laquelle structure est composée d'une pluralité de premiers éléments adhésifs (10, 20) discrets et la première structure adhésive mécanique pouvant être amenée en liaison adhésive avec une seconde structure adhésive mécanique située sur la seconde face du support (30), à l'opposé de la première face, le support comportant un premier et au moins un deuxième système de fils de chaîne, le premier système de fils de chaîne étant composé de fils élastiques et le au moins deuxième système de fils de chaîne étant composé de fils inélastiques.

2. Produit en nappe à usage médical selon la revendication 1, le produit en nappe (40) présentant une adhérence comprise entre 5 et 120 cN/cm et la proportion de la structure adhésive rapportée à la surface unilatérale du support textile (30) non étiré étant comprise dans une plage de 1 à 50 % de la surface unilatérale du support (30).

3. Produit en nappe à usage médical selon la revendication 1, le produit en nappe (40) présentant une adhérence comprise entre 5 et 120 cN/cm et les premiers éléments adhésifs (10, 20) présentant, selon une répartition discrète, une densité par unité de surface comprise entre 10² et 10⁶ éléments par mètre carré sur le produit en nappe non étiré.

4. Produit en nappe à usage médical selon la revendication 1, le produit en nappe (40) présentant une adhérence comprise entre 5 et 120 cN/cm et les premiers éléments adhésifs (10, 20) présentant, selon une répartition discrète, une densité par unité de surface comprise entre 10² à 10⁶ éléments par mètre carré sur le produit en nappe non étiré et la proportion de la structure adhésive rapportée à la surface unilatérale du support textile (30) non étiré étant comprise dans une plage de 1 à 50 % de la surface unilatérale du support (30).

5. Produit en nappe à usage médical selon l'une des revendications précédentes, le support (30) comportant un ou plusieurs fils de trame.

6. Produit en nappe à usage médical selon l'une des revendications précédentes, le support (30) comportant un non-tissé.

7. Produit en nappe à usage médical selon l'une des revendications précédentes, le produit en nappe (40) possédant une longueur L et une largeur B, le produit en nappe étant extensible de façon homogène sur l'ensemble de la longueur L.

8. Produit en nappe à usage médical selon l'une des revendications précédentes, les premiers éléments adhésifs (10, 20) étant formés par des corps moulés plats (10) ayant un rapport hauteur sur longueur de 1 à 10⁻³ ou par des particules adhésives (20) ayant un rapport hauteur sur longueur de 1 à 10⁻¹ et des dispositifs adhésifs (14) étant prévus sur les éléments adhésifs (10, 20).

9. Produit en nappe à usage médical selon l'une des revendications précédentes, les dispositifs adhésifs (14) présentant une forme de tige ou de crochet.

10. Produit en nappe à usage médical selon l'une des revendications précédentes, la seconde structure adhésive mécanique étant formée par le support (30) lui-même.

11. Produit en nappe à usage médical selon l'une des revendications précédentes, le support textile (30) étant un tissé, un tissu à mailles, un tissu tricoté, un tissu cousu-tricoté ou un non-tissé cousu-tricoté.

12. Produit en nappe à usage médical selon l'une des revendications précédentes, le produit en nappe (40) présentant un poids par unité de surface d'au moins 10 g/m² et d'au plus 350 g/m².

13. Produit en nappe à usage médical selon l'une des revendications précédentes, ce produit en nappe à usage médical étant une bande ou un bandage de compression élastique ou une bande ou un bandage de fixation élastique ou une bande ou un bandage de maintien élastique.

14. Procédé destiné à la fabrication d'un produit en nappe adhésif à usage médical selon l'une des revendications 1 à 13, les éléments adhésifs munis de dispositifs adhésifs étant fabriqués dans une première phase par le découpage de feuilles de polymère en morceaux de longueur L, de largeur B et de hauteur H et les découpes étant agencées dans une deuxième phase sur une surface d'un support textile selon une répartition discrète et étant ensuite liées et fixées au support par couture ou collage ou soudage ou compression.

15. Procédé destiné à la fabrication d'un produit en nappe adhésif à usage médical selon l'une des revendications 1 à 13, les particules adhésives munies de dispositifs adhésifs étant directement fabriquées par extrusion selon une longueur L, une largeur B et une hauteur H définies et agencées ensuite sur une surface d'un support textile selon une répartition discrète, puis liées et fixées au support par couture ou collage ou soudage ou compression.
